# EUROPÄISCHE PATENTANMELDUNG

(11) **EP 3 636 734 A1**
(43) Veröffentlichungstag der Anmeldung: **15.04.2020**
(21) Anmeldenummer: 19199435.9
(22) Anmeldetag: 25.09.2019
(51) Int. Cl.: C11D 3/12, C11D 3/37, C11D 17/04, C11D 3/00

(54) **POLYORGANOSILOXAN-BESCHICHTETER UND/ODER MIT AMORPHEN SILIZIUMDIOXIDBESCHICHTETER TEXTILER WASCHARTIKEL**

(30) Priorität: 12.10.2018 DE 102018125333
(71) Anmelder: Henkel AG & Co. KGaA, 40589 Düsseldorf (DE)
(72) Erfinder: SCHAEFER, Sascha, 40822 Mettmann (DE); NEUHAUS, Svenja, 47179 Duisburg (DE)
(74) Vertreter: Viering, Jentschura & Partner mbB Patent- und Rechtsanwälte

(57) **Zusammenfassung**

Die Erfindung betrifft einen beschichteten textilen Waschartikel, umfassend ein wasserunlösliches textiles Substrat, wobei das textile Substrat mit mindestens einem modifizierten oder unmodifizierten Polyorganosiloxan und/oder mindestens einem amorphen Siliziumdioxid beaufschlagt ist. Ferner betrifft die Erfindung die Verwendung des erfindungsgemäßen beschichteten textilen Waschartikels zum Waschen von Wäsche und/oder zur Unterbindung von Vergrauung von Wäsche, ein Verfahren zum maschinellen oder manuellen Waschen von Wäsche unter Einsatz des beschichteten textilen Waschartikels und ein Verfahren zu dessen Herstellung.

## Beschreibung

Die Erfindung betrifft einen beschichteten textilen Waschartikel, umfassend ein wasserunlösliches textiles Substrat, wobei das textile Substrat mit mindestens einem modifizierten oder unmodifizierten Polyorganosiloxan und/oder mindestens einem amorphen Siliziumdioxid beaufschlagt ist. Ferner betrifft die Erfindung die Verwendung des erfindungsgemäßen beschichteten textilen Waschartikels zum Waschen von Wäsche und/oder zur Unterbindung von Vergrauung von Wäsche, ein Verfahren zum maschinellen oder manuellen Waschen von Wäsche unter Einsatz des beschichteten textilen Waschartikels und ein Verfahren zu dessen Herstellung.

Im Hinblick auf nachhaltigere und umweltschonendere Waschprozesse gilt es mit möglichst wenigen Inhaltsstoffen eine akzeptable Reinigung zu erhalten. Viele der heute verwendeten Formulierungen für Waschmittel enthalten neben Stoffen, die die Schmutzablösung und das Halten des Schmutzes in der Waschflotte ermöglichen auch Stoffe zur Verminderung der Vergrauung, eines Prozesses, der beim Waschen von insbesondere hellen Textilien zu einer Eintrübung und damit zu einer Reduzierung der wahrgenommenen Sauberkeit der Wäsche führt. Das Halten des Schmutzes in der Waschflotte und die Unterbindung von Vergrauung ist ein wichtiges Performance-Merkmal moderner Waschsysteme.

Solche Waschsysteme sind heute für den Verbraucher in vielfältigen Angebotsformen erhältlich. Neben Pulvern und Granulaten umfasst dieses Angebot auch Flüssigkeiten, Gele oder Portionspackungen (Tabletten oder gefüllte Beutel). Insbesondere Portionspackungen erfüllten dabei den Wunsch des Verbrauchers nach vereinfachter Dosierung. Waschmitteltabletten oder mit flüssigen und/oder festen Waschmitteln gefüllte Beutel weisen jedoch auch eine Reihe von Nachteilen auf. Insbesondere tablettierte Angebotsformen zeichnen sich aufgrund ihrer hohen Verdichtung häufig durch einen verzögerten Zerfall und damit eine verzögerte Freisetzung ihrer Inhaltsstoffe aus. Daher hat der Schmutz, der in der Waschflotte dispergiert ist, mehr Zeit sich auf einem Gewebe abzusondern.

Bei mit flüssigen Waschmitteln gefüllten Portionsbeuteln besteht der Nachteil, dass inkompatible Inhaltsstoffe nicht miteinander formuliert werden können. Oftmals lösen sich auch die verwendeten Hüllmaterialien (beispielsweise Polyvinylalkohol) nicht rechtzeitig auf und verbleiben als Rückstände auf der gewaschenen Wäsche. Ebenfalls gelangen die gelösten polymeren Bestandteile mit der Waschflotte in die Umwelt, was vermieden werden sollte.

Die Aufgabe der vorliegenden Erfindung war es deshalb eine einfach zu handhabende Angebotsform für ein Waschmittel bereitzustellen, welches verbesserte Eigenschaften aufweist, insbesondere vor Vergrauen schützt und eines oder mehrere der vorstehenden Nachteile überkommt.

Die Erfinder der vorliegenden Erfindung haben überraschend gefunden, dass dies mit dem beschichteten textilen Waschartikel der vorliegenden Erfindung möglich ist. Dabei wurde gefunden, dass sich Polyorganosiloxane oder amorphe Siliziumdioxide eignen, um mit einem textilen Substrat kompatibel zu sein und um die Vergrauung der Wäsche zu verhindern oder zu reduzieren. Ebenfalls vermeidet der textile Waschartikel einen erhöhten Eintrag von Polymeren in die Waschflotte, da dieser nach dem Waschprozess als Substrat/Tuch im Hausmüll entsorgt wird.

Die vorliegende Erfindung betrifft in einem ersten Aspekt einen beschichteten textilen Waschartikel, umfassend ein wasserunlösliches textiles Substrat, wobei das textile Substrat mit mindestens einem modifizierten oder unmodifizierten Polyorganosiloxan und/oder mindestens einem amorphen Siliziumdioxid beaufschlagt ist.

Des Weiteren betrifft die Erfindung in einem zweiten Aspekt die Verwendung des beschichteten textilen Waschartikels gemäß der vorliegenden Erfindung zum Waschen von Wäsche und/oder zur Unterbindung von Vergrauung von Wäsche.

In einem dritten Aspekt betrifft die vorliegende Erfindung ein Verfahren zum maschinellen oder manuellen Waschen von Wäsche unter Einsatz eines textilen Waschartikels der vorliegenden Erfindung.

Schließlich betrifft die Erfindung in einem vierten Aspekt ein Verfahren zur Herstellung eines beschichteten textilen Waschartikels gemäß der vorliegenden Erfindung, umfassend die Schritte:
(i) Bereitstellen eines wasserunlöslichen textilen Substrats,
(ii) Beaufschlagen des textilen Substrats mit mindestens einem modifizierten oder unmodifizierten Polyorganosiloxan und/oder mit mindestens einem amorphen Siliziumdioxid und gegebenenfalls einer bei Erwärmung über 30°C fließfähigen Waschmittelzusammensetzung und gegebenenfalls einer weiteren Zusammensetzung auf mindestens einer Zone des textilen Substrats und
(iii) Abkühlen und Erhärten der Komponenten auf dem textilen Substrat.

"Mindestens ein", wie hierin verwendet, bezieht sich auf 1 oder mehr, beispielsweise 2, 3, 4, 5, 6, 7, 8, 9 oder mehr. Im Zusammenhang mit der hierin beschriebenen Erfindung bezieht sich diese Angabe nicht auf die absolute Menge oder Anzahl eines Moleküls oder Bestandteils, sondern auf die Art des Bestandteils. "Mindestens ein Polyorganosiloxan" bedeutet daher beispielsweise, dass mindestens eine Art von Polyorganosiloxanen vorliegt, aber auch zwei oder mehr verschiedene Arten von Polyorganosiloxanen enthalten sein können. Mindestens ein bezieht sich dabei nicht auf die Menge an Polyorganosiloxan-Molekülen, die in der Zusammensetzung vorhanden sind.

Zahlenwerte, die hierin ohne Dezimalstellen angegeben sind, beziehen sich jeweils auf den vollen angegebenen Wert mit einer Dezimalstelle. So steht beispielsweise "99 %" für "99,0 %".

Der Ausdrücke "ungefähr" oder "etwa", in Zusammenhang mit einem Zahlenwert, bezieht sich auf eine Varianz von ±10 % bezogen auf den angegebenen Zahlenwert, bevorzugt ±5 %, besonders bevorzugt ±1 %.

Alle Prozentangaben sind, sofern nicht anders angegeben, Gewichts-% (Gew.-%). Numerische Bereiche, die in dem Format von "x bis y" angegeben sind, schließen die genannten Werte "x" und "y" ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

Die vorstehend beschriebenen und weiteren Aspekte, Ausführungsformen, Merkmale und Vorteile der Erfindung werden für den Fachmann aus dem Studium der nachfolgenden detaillierten Beschreibung und Ansprüche ersichtlich. Dabei kann jedes Merkmal aus einer Ausführungsform der Erfindung in jede andere Ausführungsform der Erfindung eingesetzt werden. Ferner ist es selbstverständlich, dass die hierin enthaltenen Beispiele die Erfindung beschreiben und veranschaulichen sollen, diese aber nicht einschränken und insbesondere die Erfindung nicht auf diese Beispiele beschränkt ist.

Die nachfolgend dargestellten Sachverhalte, Gegenstände und Ausführungsformen, die für den beschichteten textilen Waschartikel beschrieben werden, sind auch auf das erfindungsgemäße Herstellungsverfahren, Verfahren zum maschinellen oder manuellen Waschen von Wäsche und der Verwendung anwendbar.

Der beschichtete textile Waschartikel umfasst ein wasserunlösliches textiles Substrat, wobei das textile Substrat mit mindestens einem modifizierten oder unmodifizierten Polyorganosiloxan beaufschlagt ist.

In verschiedenen Ausführungsformen ist das mindestens eine modifizierte oder unmodifizierte Polyorganosiloxan ausgewählt aus der Gruppe von Polyalkylsiloxanen, Fluorsiliconen, Ether modifizierten Siliconen und Polyether modifizierten Siliconen.

Bevorzugte unmodifizierte Polyalkylsiloxane weisen eine Struktur nach Formel I auf: 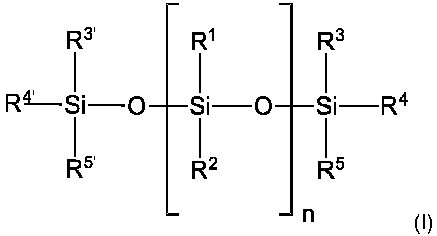
wobei R¹ und R² unabhängig voneinander ein C1-C10 Alkylrest, bevorzugt Ethyl, Methyl oder Propyl, stärker bevorzugt Methyl, sind,
R³ bis R⁵ und R^{3'} bis R^{5'} unabhängig voneinander ein C1-10 Alkylrest oder ein Halogenatom, insbesondere -F; bevorzugt Ethyl, Methyl oder Propyl, stärker bevorzugt Methyl, sind, und
n eine ganze Zahl von 10 bis 1800, bevorzugt 20 bis 1600, stärker bevorzugt 150 bis 1500, am stärksten bevorzugt 300 bis 1350 ist.

Alternative bevorzugte unmodifizierte Polyalkylsiloxane sind cyclische Polyalkylsiloxane mit einer Struktur nach Formel Ia: 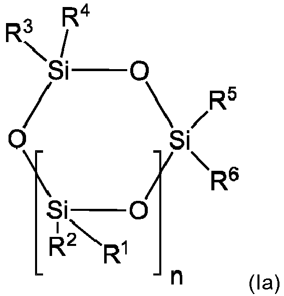
wobei R¹ bis R⁶ unabhängig voneinander ein C1-C10 Alkylrest, bevorzugt Ethyl, Methyl oder Propyl, stärker bevorzugt Methyl, sind, und
n eine ganze Zahl von 1 bis 1000, bevorzugt 2 bis 100, stärker bevorzugt 2 bis 50, am stärksten bevorzugt 2 oder 3 ist. Insbesondere bevorzugt ist Dodecamethylcyclohexasiloxan.

Bevorzugte Fluorsilicone weisen eine Struktur nach Formel II auf: 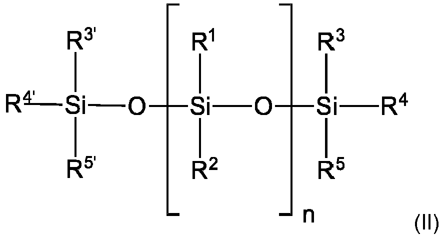 wobei R¹ und R² unabhängig voneinander ein C1-C10 Alkylrest, bevorzugt Ethyl, Methyl oder Propyl, stärker bevorzugt Methyl, oder -(CH₂)ₘCFₚH₃₋ₚ, mit m = 1 bis 10, p = 1-3, sind, R³ bis R⁵ und R^{3'} bis R^{5'} unabhängig voneinander ein C1-10 Alkylrest oder ein Halogenatom, insbesondere -F; bevorzugt Ethyl, Methyl oder Propyl, stärker bevorzugt Methyl, sind, und n eine ganze Zahl von 10 bis 1800, bevorzugt 20 bis 1600, stärker bevorzugt 150 bis 1500, am stärksten bevorzugt 300 bis 1350 ist, mit der Maßgabe, dass in mindestens einer Einheit n, bevorzugt 2 bis 1500, stärker bevorzugt 5 bis 1000 Einheiten, ein -(CH₂)ₘCFₚH₃₋ₚ Rest enthalten ist.

Bevorzugte mit Ether oder Polyether modifizierte Polyalkylsiloxane weisen eine Struktur nach Formel III auf: 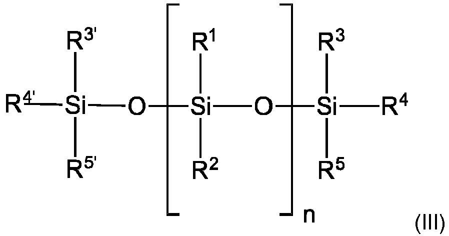 wobei R¹ und R² unabhängig voneinander ein C1-C10 Alkylrest, bevorzugt Ethyl, Methyl oder Propyl, stärker bevorzugt Methyl, oder -(CH₂)ₘO(CₚH₂ₚO)_{q}H, mit m = 1 bis 10, p = 1-5, bevorzugt 2 oder 3, q = 1 bis 1500, bevorzugt 3 bis 150, sind, R³ bis R⁵ und R^{3'} bis R^{5'} unabhängig voneinander ein C1-10 Alkylrest oder ein Halogenatom, insbesondere -F; bevorzugt Ethyl, Methyl oder Propyl, stärker bevorzugt Methyl, sind, und n eine ganze Zahl von 10 bis 1800, bevorzugt 20 bis 1600, stärker bevorzugt 150 bis 1500, am stärksten bevorzugt 300 bis 1350 ist, mit der Maßgabe, dass in mindestens einer Einheit n, bevorzugt 2 bis 1500, stärker bevorzugt 5 bis 1000 Einheiten, ein -(CH₂)ₘO(CₚH₂ₚO)_{q}H Rest enthalten ist.

Geeignete Polyorganosiloxane sind beispielsweise kommerziell von der Firma Dow Corning erhältlich, zum Beispiel unter dem Handelsnamen AC-8066.

In verschiedenen Ausführungsformen ist mindestens ein amorphes Siliziumdioxid enthalten.

Generell sind alle dem Fachmann bekannten amorphen Siliziumdioxide geeignet. "Amorphes Silziumdioxid" ist nichtkristallines oder niedrigkristallines Siliziumdioxid. Amorphes Siliziumdioxid weist keine Fernordnung auf und unterscheidet sich somit von kristallinem Siliziumdioxid wie Quarz. Amorphes Siliziumdioxid umfasst Quarzglas aus amorphem Siliziumdioxid. Dieses wird durch Schmelzen von kristallinem Siliziumdioxid (natürlich vorkommendem Quarz) zu einer nichtkristallinen Form hergestellt. Alternativ kann synthetisches Quarzglas durch Pyrolyse von Siliziumtetrachlorid oder verdampftem Quarz hergestellt werden, um winzige Tröpfchen aus amorphem Quarzglas zu bilden, die zu einer artikulierten Partikelstruktur verschmelzen.

Eine solche Form von synthetischem Quarzglas ist auch unter dem Begriff pyrogene Kieselsäure bekannt. Amorphes Siliziumdioxid kann auch aus der Lösung ausgefällt werden, um kleine poröse Teilchen zu bilden, die in Ketten miteinander verschmelzen können. Eine solche Form von Kieselsäure ist als Kieselgel bekannt, das zur Bildung von Kieselsäureaerogelen verwendet werden kann. Das Einstellen des pH-Werts der Lösung kann die Partikel getrennt halten, um größere Einzelpartikel zu bilden, die üblicherweise als gefälltes Siliziumdioxid oder Siliziumdioxidsole bezeichnet werden, die alle Formen von amorphem Siliziumdioxid sind. In verschiedenen Ausführungsformen ist das amorphe Siliziumdioxid bei 22 ° C und 1 bar fest. In verschiedenen Ausführungsformen ist die amorphe Kieselsäure ausgewählt aus der Gruppe bestehend aus Kieselsäureaerogelen, Kieselgel, pyrogener Kieselsäure und Kombinationen davon.

In verschiedenen Ausführungsformen, insbesondere wenn es sich um pyrogene Kieselsäure handelt, kann die amorphe Kieselsäure mit einer Oberflächenbehandlung behandelt werden. Solche Oberflächenbehandlungen umfassen, ohne darauf beschränkt zu sein, Polydimethylsiloxanbeschichtungen und Vinylalkoxysilane. Im Allgemeinen machen die Substanzen, welche zur Oberflächenbehandlung verwendet werden nicht mehr als 10 Gewichtsprozent ("Gew.-%") des gesamten behandelten amorphen Siliziumdioxids aus und können im Allgemeinen weniger als 5 Gew .-% betragen. In einer bevorzugten Ausführungsform wird mindestens ein behandeltes amorphes Siliziumdioxid, insbesondere mindestens eine behandelte pyrogene Kieselsäure, verwendet.

In einer bevorzugten Ausführungsform liegt sowohl mindestens ein modifiziertes oder unmodifiziertes Polyorganosiloxan und mindestens ein amorphes Siliziumdioxid vor. Bevorzugt liegt dabei mindestens ein cyclisches Polyalkylsiloxan nach Formel (la) wie vorstehend definiert und/oder mindestens ein behandeltes amorphes Siliziumdioxid vor. In einer bevorzugten Ausführungsform ist das mindestens eine modifizierte oder unmodifizierte Polyorganosiloxan zu dem mindestens einen amorphen Siliziumdioxid in einem Gewichtsverhältnis von 0,1 : 99 bis 10 : 90, bevorzugt 0,8 : 99,2 bis 1,2 : 98,2, enthalten.

Der erfindungsgemäße Waschartikel kann dabei bevorzugt permanent mit dem mindestens einem modifizierten oder unmodifizierten Polyorganosiloxan und/oder mindestens einem amorphen Siliziumdioxid ausgerüstet sein.

Unter einer permanenten Ausrüstung wird im Rahmen dieser Erfindung eine dauerhafte chemische und/oder physikalische Veränderung des wasserunlöslichen Substrats verstanden, die diesem bestimmte Gebrauchseigenschaften, im vorliegenden Fall vergrauungsinhibierenden Eigenschaften, vermittelt. Diese Veränderung kann an den Fasern, welche später das wasserunlösliche textile Substrat bilden, oder an dem wasserunlöslichen textilen Substrat vorgenommen werden.

Die Ausrüstung des textilen Substrats mit vergrauungsinhibierenden Eigenschaften erfolgt durch Aufbringen von mindestens einem modifizierten oder unmodifizierten Polyorganosiloxans und/oder von mindestens einem amorphen Siliziumdioxid. Dieses ist in einer Ausführungsform der Erfindung faserreaktiv und geht mit den Fasern des wasserunlöslichen textilen Substrats eine ionische oder kovalente Bindung, bevorzugt jedoch eine van-der-Waals Bindung ein. Die Ausrüstung der Fasern kann beispielsweise mittels eines so genannten Aufziehverfahrens erfolgen, bei dem die Fasern mit einer Anwendungsflotte, das heißt beispielsweise mit einer Lösung, Dispersion oder ähnlichem des Polyorganosiloxans und/oder amorphen Siliziumdioxids, behandelt werden. Während der Behandlung zieht das oder die eingesetzte(n) Polyorganosiloxan(e) und/oder amorphe(n) Siliziumdioxid(e) aus der Anwendungsflotte auf die behandelten Fasern gleichmäßig auf. Alternativ kann das wasserunlösliche textile Substrat in Form eines Vlieses oder eines Gewebes in die Anwendungsflotte getaucht oder mit dieser bedruckt oder besprüht werden. Das mindestens eine Polyorganosiloxan und/oder mindestens eine amorphe Siliziumdioxid kann vor, nach oder gemeinsam mit der gegebenenfalls vorhandenen Waschmittelzusammensetzung und/oder weiteren Zusammensetzung aufgebracht werden.

In weiteren verschiedenen Ausführungsformen weist das mindestens eine modifizierte oder unmodifizierte Polyorganosiloxan ein Molekulargewicht von 150 bis 50.000 g/mol oder 1.000 bis 35.000 g/mol oder 2.500 bis 25.000 g/mol auf. Das Molekulargewicht wird dabei bevorzugt mittels Gelpermeationschromatographie unter der Verwendung von Polystyrolstandards bestimmt.

In bevorzugten Ausführungsformen ist das mindestens eine modifizierte oder unmodifizierte Polyorganosiloxan in 0,001 bis 50 Gew.-% oder 1,0 bis 10,0 Gew.-%, bezogen auf das Gesamtgewicht des beschichteten textilen Waschartikels, enthalten. In besonders bevorzugten Ausführungsformen beträgt das Gesamtgewicht an modifizierten oder unmodifizierten Polyorganosiloxanen 0,001 bis 50 Gew.-% oder 1,0 bis 10,0 Gew.-%, bezogen auf das Gesamtgewicht des beschichteten textilen Waschartikels.

In bevorzugten Ausführungsformen ist das mindestens eine amorphe Siliziumdioxid in 0,001 bis 50 Gew.-% oder 1,0 bis 10,0 Gew.-%, bezogen auf das Gesamtgewicht des beschichteten textilen Waschartikels, enthalten. In besonders bevorzugten Ausführungsformen beträgt das Gesamtgewicht an amorphen Siliziumdioxid 0,001 bis 50 Gew.-% oder 1,0 bis 10,0 Gew.-%, bezogen auf das Gesamtgewicht des beschichteten textilen Waschartikels.

Es ist bevorzugt, dass das textile Substrat ein Gewebe oder ein Vlies ist. Gewebe und Vliese weisen eine hohe Strapazierfähigkeit und gute Beständigkeit auf, so dass diese textilen Substrate den hohen mechanischen Belastungen während des Waschvorgangs, insbesondere in einer Waschmaschine, standhalten. In bevorzugten Ausführungsformen besteht das textile Substrat aus Cellulose (beispielsweise Baumwolle), Polyester, Polyethylen, Polypropylen, Polyethylenterephthalat, Polybutylenterephthalat, Rayon, Polyacryl, Polymilchsäure, Lyocell, Viskose oder Nylon oder Mischungen davon. In einer besonders bevorzugten Ausführungsform enthält das textile Substrat wenigstens zwei verschiedene Materialien, wobei ein Material nicht-modifiziertes Polypropylen ist. Polypropylenfasern können Fett binden und wirken so zusätzlich einer Vergrauung entgegen.

In einer weiteren, bevorzugten Ausführungsform liegt auf dem Substrat wenigstens eine weitere Zusammensetzung verfestigt vor. Bevorzugt umfasst das Substrat eine Waschmittelzusammensetzung. Neben der Waschmittelzusammensetzung kann eine weitere, beispielsweise eine Textil- und/oder Hautpflegende Zusammensetzung, auf dem textilen Substrat vorliegen. Der Waschartikel kann so um eine weitere Funktionalität erweitert werden. Die Zusammensetzungen liegen bevorzugt verfestigt vor.

Dabei kann es bevorzugt sein, dass die Waschmittelzusammensetzung eine Farbe aufweist, die von der Farbe der weiteren Zusammensetzung verschieden ist, um den Verbrauchern auch visuell zu zeigen, dass der beschichtete textile Waschartikel mehrere Funktionalitäten besitzt.

Es kann bevorzugt sein, dass die Waschmittelzusammensetzung und die weitere Zusammensetzung nebeneinander auf dem textilen Substrat angeordnet sind. In einer anderen, bevorzugten Ausführungsform sind die Waschmittelzusammensetzung und die weitere Zusammensetzung zumindest teilweise überlappend auf dem textilen Substrat angeordnet.

Durch die unterschiedliche Anordnung der Zusammensetzungen können beispielsweise für die jeweiligen Zusammensetzungen unterschiedliche Auflöseprofile und dadurch Freisetzungsprofile der enthaltenen Inhaltsstoffe eingestellt werden.

Bevorzugt können die Fasern oder das textile Substrat zusätzlich mit einer Kupplungskomponente, beispielsweise Epichlorhydrin, und anschließend mit einer basischen Stickstoffverbindung umgesetzt werden. Durch anschließende Quaternisierung wird eine Schmutz-fangende Ausrüstung der Fasern erhalten.

In einer weiteren bevorzugten Ausführungsform der Erfindung werden die Fasern oder das textile Substrat zusätzlich mit anionischen Gruppen, beispielsweise Carboxylgruppen und/oder Sulfonatgruppen, versehen und anschließend mit den Schmutz-fangenden Verbindungen umgesetzt.

Die Schmutz-fangende Verbindung weist vorzugsweise eine kationische Ladung auf. Insbesondere ist es bevorzugt, dass die Schmutz-fangende Verbindung ein kationisches Polymer ist. Nichtionische Schmutz-fangende Verbindungen können beispielsweise mit Hilfe von kationischen Kupplungsgruppen in kationisch geladene Verbindungen überführt werden.

Es ist bevorzugt, dass die Schmutz-fangende Verbindung ausgewählt ist aus der Gruppe der polyfunktionellen quaternären Ammoniumverbindungen, wie beispielsweise Polyquaternium-6, Polyquaternium-10 und kationische Polygalactomannan-Derivate, der Acrylpolymere, wie beispielsweise superabsorbierende Polyacrylate, der Polyester und/oder der heterocyclischen Polymere, wie beispielsweise Polyvinylpyrrolidon, Polyamin-N-oxid-Polymere, Polyvinylimidazol sowie Copolymere aus Vinylpyrrolidon, Vinylimidazol, Vinyloxazolidon, Vinylpyridin und Vinylpyridin-N-oxid.

Zum Einfangen von unterschiedlichen Schmutzarten kann es bevorzugt sein, dass das textile Substrat mit zwei oder mehr Schmutz-fangenden Verbindungen ausgerüstet ist.

Die Ausmaße des als Flächengebilde vorliegenden textilen Substrats sollten eine leichte Handhabbarkeit des Waschartikels zulassen und zum Beispiel 10 cm × 10 cm oder 20 cm × 20 cm betragen, obwohl auch andere Größen möglich sind. Das textile Substrat und damit der Waschartikel kann von beliebiger Flächenform, beispielsweise quadratisch, rechteckig, dreieckig, rund, oval, herzförmig, buchstaben- oder wortförmig oder der Wiedergabe einer Marke entsprechend sein. Das textile Substrat kann jede beliebige Farbe aufweisen oder im Wesentlichen weiß sein; seine Oberfläche kann im Wesentlichen glatt oder herstellungsbedingt texturiert sein. Vorzugsweise weist das textile Substrat und damit der Waschartikel eine Oberfläche (bezogen auf eine Seite) von 40 cm² bis 800 cm² auf. In einer bevorzugten Ausführungsform der Erfindung ist das textile Substrat zumindest an Teilen seiner Oberflächen aufgeraut und weist so eine Oberflächenvergrößerung auf, wodurch eine bessere und stabilere Aufziehbarkeit der Polyorganosiloxane erreicht wird. Aufgrund der Oberflächenvergrößerung kann eine größere Menge der Polyorganosiloxane auf- bzw. angebracht werden, so dass insgesamt eine größere Anzahl an Bindungsstellen für zu fixierende vergrauend wirkende Moleküle auf bzw. an dem textilen Substrat vorliegt. Dies erhöht die Leistungsfähigkeit des beschichteten textilen Waschartikels in Bezug auf seine vergrauungsinhibierenden Eigenschaften.

In einer weiteren bevorzugten Ausführungsform weist das textile Substrat zusätzlich zu der vergrauungsinhibierenden Ausrüstung eine antimikrobielle Ausrüstung auf. Die antimikrobielle Ausrüstung kann beispielsweise durch Einarbeiten von Silberfäden während der Herstellung des textilen Substrats oder durch Aufbringen von Silberverbindungen auf bzw. an dem textilen Substrat, wie beispielsweise SILVERPLUS® (ex Rudolf Chemie) erfolgen.

Auf dem textilen Substrat befindet sich bevorzugt eine verfestigte Waschmittelzusammensetzung.

Eine auf das textile Substrat aufzubringende Waschmittelzusammensetzung enthält, insbesondere wenn sie als Schmelze aufgebracht werden soll, bei Raumtemperatur feste beziehungsweise wachsartige, bei höherer Temperatur schmelzbare Bestandteile, zu denen insbesondere Tenside und organische Polymere zu rechnen sind. Außerdem können in der Waschmittelzusammensetzung für Wasch- beziehungsweise Wäschenachbehandlungsmittel übliche Inhaltsstoffe enthalten sein.

Eine erfindungsgemäß auf das textile Substrat aufzubringende Waschmittelzusammensetzung kann insbesondere Buildersubstanzen, Tenside, Bleichmittel, Bleichkatalysatoren, Bleichaktivatoren, Enzyme, Sequestrierungsmittel, Elektrolyte, pH-Regulatoren, Polymere mit Spezialeffekten, wie Soil-Release-Polymere, Farbübertragungsinhibitoren, knitterreduzierende Wirkstoffe und formerhaltende Wirkstoffe, und weitere Hilfsstoffe, wie optische Aufheller, Fungizide, Antioxidantien, UV-Absorber Schaumregulatoren, Bleichaktivatoren, Farbstoffe, antimikrobielle Wirkstoffe und Duftstoffe enthalten.

Eine bevorzugte Waschmittelzusammensetzungen kann ein oder mehrere Tenside enthalten, wobei insbesondere anionische Tenside, nichtionische Tenside und deren Gemische, aber auch kationische Tenside in Frage kommen. Geeignete nichtionische Tenside sind insbesondere Alkylglykoside und Ethoxylierungs- und/oder Propoxylierungsrodukte von Alkylglykosiden oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkyl-aminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar.

Geeignete anionische Tenside sind insbesondere Seifen und solche, die Sulfat- oder Sulfonat-Gruppen mit bevorzugt Alkaliionen als Kationen enthalten. Verwendbare Seifen sind bevorzugt die Alkalisalze der gesättigten oder ungesättigten Fettsäuren mit 12 bis 18 C-Atomen. Derartige Fettsäuren können auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 C-Atomen und die Sulfatierungsprodukte der genannten nichtionischen Tenside mit niedrigem Ethoxylierungsgrad. Zu den verwendbaren Tensiden vom Sulfonat-Typ gehören lineare Alkylbenzolsulfonate mit 9 bis 14 C-Atomen im Alkylteil, Alkansulfonate mit 12 bis 18 C-Atomen, sowie Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen. Derartige Tenside sind in der erfindungsgemäß auf das textile Substrat aufzubringenden Waschmittelzusammensetzung in Mengen von vorzugsweise 5 Gew.-% bis 50 Gew.-%, insbesondere von 8 Gew.-% bis 30 Gew.-%, bezogen auf das Gesamtgewicht der Waschmittelzusammensetzung enthalten.

Die Waschmittelzusammensetzung kann auch kationische Tenside enthalten. Kationische Tenside werden vorzugsweise unter den Esterquats und/oder den quaternären Ammoniumverbindungen (QAV) gemäß der allgemeinen Formel (R^{I})(R^{II})(R^{III})(R^{IV})N⁺ X⁻ ausgewählt, in der R^{I} bis R^{IV} für gleiche oder verschiedene C1-22-Alkylreste, C7-28-Arylalkylreste oder heterozyklische Reste stehen, wobei zwei oder im Falle einer aromatischen Einbindung wie im Pyridin sogar drei Reste gemeinsam mit dem Stickstoffatom den Heterozyklus, z. B. eine Pyridinium- oder Imidazoliniumverbindung, bilden, und X⁻ für Halogenidionen, Sulfationen, Hydroxidionen oder ähnliche Anionen steht. In Frage kommende QAV sind beispielsweise Benzalkoniumchlorid (N Alkyl-N,N-dimethyl-benzylammoniumchlorid), Benzalkon B (m,p-Dichlorbenzyl-dimethyl-C12-alkylammoniumchlorid), Benzoxoniumchlorid (Benzyl-dodecyl-bis-(2-hydroxyethyl)-ammoniumchlorid), Cetrimoniumbromid (N-Hexadecyl-N,N-trimethyl-ammoniumbromid), Benzetoniumchlorid (N,N-Dimethyl-N-[2-[2-[p-(1,1,3,3-tetramethylbutyl)phenoxy]-ethoxy]-ethyl]benzylammoniumchlorid), Dialkyldimethylammoniumchloride wie Di-n-decyldimethylammoniumchlorid, Didecyldimethylammoniumbromid, Dioctyldimethylammoniumchlorid, 1-Cetylpyridiniumchlorid und Thiazolinjodid sowie deren Mischungen. Bevorzugte QAV sind die Benzalkoniumchloride mit C8-C18-Alkylresten, insbesondere C12-C14-Alkyl-benzyl-dimethylammoniumchlorid.

Geeignete Beispiele für Esterquats sind beispielsweise in den Formeln (I) und (II) gezeigt:

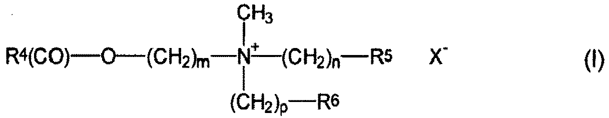

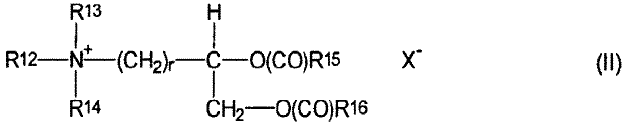

In Formel (I) steht R⁴ für einen aliphatischen Alk(en)ylrest mit 12 bis 22 Kohlenstoffatomen mit 0, 1, 2 oder 3 Doppelbindungen und/oder gegebenenfalls mit Substituenten; R⁵ steht für H, OH oder O(CO)R⁷, R⁶ steht unabhängig von R⁵ für H, OH oder O(CO)R⁸, wobei R⁷ und R⁸ unabhängig voneinander jeweils für einen aliphatischen Alk(en)ylrest mit 12 bis 22 Kohlenstoffatomen mit 0, 1, 2 oder 3 Doppelbindungen steht. m, n und p können jeweils unabhängig voneinander den Wert 1, 2 oder 3 haben. X- kann entweder ein Halogenid-, Methosulfat-, Methophosphat- oder Phosphation sowie Mischungen aus diesen Anionen sein. Bevorzugt sind Verbindungen, bei denen R⁵ die Gruppe O(CO)R⁷ darstellt. Besonders bevorzugt sind Verbindungen, bei denen R⁵ die Gruppe O(CO)R⁷ darstellt und R⁴ und R⁷ Alk(en)ylreste mit 16 bis 18 Kohlenstoffatomen sind. Insbesondere bevorzugt sind Verbindungen, bei denen R⁶ zudem für OH steht. In Formel (II) steht R¹², R¹³ und R¹⁴ unabhängig voneinander für eine C1-4-Alkyl-, Alkenyl- oder Hydroxyalkylgruppe, R¹⁵ und R¹⁶ jeweils unabhängig ausgewählt eine C8-28-Alkylgruppe, X⁻ ist ein Anion und r ist eine Zahl zwischen 0 und 5 ist. Beispiele für brauchbare Esterquats umfassen Methyl-N-(2-hydroxyethyl)-N,N-di(talgacyloxyethyl)ammoniummethosulfat, Bis-(palmitoyloxyethyl)-hydroxyethyl-methyl-ammonium-methosulfat, 1,2-Bis-[talgacyloxy]-3-trimethylammoniumpropanchlorid, Methyl-N,N-bis(stearoyloxyethyl)-N-(2-hydroxyethyl)ammonium-methosulfat oder N,N-Dimethyl-N,N-di(talgacyloxyethyl)ammoniummethosulfat.

Es ist allerdings bevorzugt, dass die kationischen Tenside, insbesondere die Esterquats, falls vorhanden, nicht in der Waschmittelzusammensetzung vorliegen, sondern in einer weiteren Zusammensetzung.

Geeignete Bleichmittel für den Einsatz in erfindungsgemäßen Waschartikeln umfassen insbesondere organische Persäuren beziehungsweise persaure Salze organischer Säuren, wie Phthalimidopercapronsäure, Perbenzoesäure oder Salze der Diperdodecandisäure, und unter den Waschbedingungen Wasserstoffperoxid abgebende anorganische Salze, zu denen Alkaliperborat, Alkalipercarbonat, -persilikat und/oder -persulfat wie Caroat gehören, in Betracht. Die Persauerstoffverbindungen können in Form von Pulvern oder Granulaten verwendet werden, die auch in im Prinzip bekannter Weise umhüllt sein können. Der Zusatz geringer Mengen bekannter Bleichmittelstabilisatoren wie beispielsweise von Phosphonaten, Borsten beziehungsweise Metaboraten und Metasilikaten sowie Magnesiumsalzen wie Magnesiumsulfat kann zweckdienlich sein. Eine auf das textile Substrat aufzubringende bleichmittelhaltige Waschmittelzusammensetzung enthält in einer bevorzugten Ausführungsform 15 Gew.-% bis 50 Gew.-%, insbesondere 18 Gew.-% bis 35 Gew.-% persauerstoffhaltiges Bleichmittel, insbesondere Alkalipercarbonat.

Zusätzlich zum Bleichmittel kann, entweder als Teil der bleichmittelhaltigen Waschmittelzusammensetzung oder als Teil einer weiteren Zusammensetzung, Bleichaktivator eingearbeitet werden. Als Bleichaktivatoren können insbesondere Verbindungen, die unter Perhydrolysebedingungen aromatische oder aliphatische Peroxocarbonsäuren ergeben, eingesetzt werden. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, sowie acylierte mehrwertige Alkohole, insbesondere Triacetin, Ethylenglykoldiacetat und 2,5-Diacetoxy-2,5-dihydrofuran. Auch unter Perhydrolysebedingungen Perimidsäuren bildende Nitrile, beispielsweise Ammoniumgruppen tragende Acetonitrile, können eingesetzt werden.

Zusätzlich zu den konventionellen Bleichaktivatoren oder an deren Stelle können auch so genannte Bleichkatalysatoren eingearbeitet werden. Bei diesen Stoffen handelt es sich um bleichverstärkende Übergangsmetallsalze beziehungsweise Übergangsmetallkomplexe wie beispielsweise Mn-, Fe-, Co-, Ru- oder Mo-Salenkomplexe oder -carbonylkomplexe. Auch Mn-, Fe-, Co-, Ru-, Mo-, Ti-, V- und Cu-Komplexe mit stickstoffhaltigen Tripod-Liganden sowie Co-, Fe-, Cu- und Ru-Amminkomplexe sind als Bleichkatalysatoren verwendbar. Zu den bevorzugten Bleichkatalysatoren gehören Komplexe mit Triazacyclononan-Liganden, die gegebenenfalls auch ein- oder mehrfach Alkylsubstituiert sein können, beispielsweise 1,4,7-Trimethyl-1,4,7-triazacyclononan.

Der beschichtete textile Waschartikel kann bevorzugt mit Mikrokapseln, insbesondere Kern-Hülle-Mikrokapseln, welche einen Wirkstoff enthalten, beaufschlagt sein. Die Mikrokapseln können direkt auf dem textilen Substrat mittels eines Klebstoffs appliziert werden oder durch die Waschmittel- oder zusätzliche Zusammensetzung eingebracht werden. Bevorzugt ist der Wirkstoff, der in den Mikrokapseln enthalten ist ein Duftstoff, ein Enzym oder ein Bleichmittel, insbesondere bevorzugt ein Duftstoff. Dabei können alle dem Fachmann bekannten Duftstoffe eingesetzt werden. Bei der Mikrokapsel handelt es sich bevorzugt um eine Melamin, Acrylat oder Polyvinylalkohol basierte Mikrokapsel.

In verschiedenen Ausführungsformen weist der beschichtete textile Waschartikel textile Substrat Fasern auf, die mit einer Wirkstoff-haltigen, wasserlöslichen Beschichtung versehen sind. In weiteren verschiedenen Ausführungsformen ist die wasserlösliche Beschichtung aus einem schichtbildenden Material gebildet, welches ausgewählt ist aus der Gruppe umfassend partikuläre Sole und wasserlösliche Polymere. Als wasserlösliche Polymere sind insbesondere Polyvinylalkohole geeignet.

Durch das Vorhandensein einer weiteren Zusammensetzung, welche verfestigt auf dem textilen Substrat vorliegt, können dem Waschartikel weitere Funktionalitäten vermittelt werden. So kann in dem Fall, in dem die verfestigte weitere Zusammensetzung einen Wäschepflegewirkstoff, wie beispielsweise eine Textil-weichmachende Verbindung und/oder einen im Wäschetrockner freisetzbaren Duftstoff, einen höheren Erweichungspunkt als die verfestigte Waschmittelzusammensetzung aufweist und sich nicht im Waschvorgang auflöst, der Waschartikel nach dem Waschvorgang zusammen mit der gewaschenen Wäsche in einen automatischen Wäschetrockner überführt werden. Dort kann dann die weitere Zusammensetzung ihre Wäschepflegende Wirkung entfalten.

In einer bevorzugten Ausführungsform ist die weitere Zusammensetzung eine Textil- und/oder Hautpflegende Zusammensetzung.

Unter einer Textil-pflegenden Zusammensetzung wird in diesem Zusammenhang jede Zusammensetzung verstanden, die damit behandelten textilen Flächengebilden einen vorteilhaften Effekt vermittelt, wie beispielsweise einen Textil-weichmachenden Effekt, Knitterfestigkeit, Duft bzw. die schädliche oder negative Effekte, die beim Reinigen und/oder Konditionieren und/oder Tragen auftreten können, wie beispielsweise Verblassen, usw., reduziert.

Die Textil-pflegende Zusammensetzung ist vorzugsweise eine Textil-weichmachende Zusammensetzung und enthält beispielsweise einen Esterquat, einen Textilweichmachenden Ton, ein kationisches Polymer oder eine Mischung aus diesen Textilweichmachenden Verbindungen. Weitere geeignete Textil-pflegende Verbindungen umfassen beispielsweise Soil-Release-Polymere, Einlaufverhinderer, Knitterschutzmittel, Farbübertragungsinhibitoren, antimikrobiellen Wirkstoffe, Germizide, Fungizide, Antioxidantien, Bügelhilfsmittel, Phobier- und Imprägniermittel sowie Duftstoffe.

Unter einer hautpflegenden Zusammensetzung wird eine Zusammensetzung mit einer hautpflegenden Verbindung verstanden, wobei letztere bei Kontakt eines Textils mit dem Waschmittel auf das Textil aufzieht und bei Kontakt des Textils mit Haut der Haut einen Vorteil verleiht verglichen mit einem Textil, welches nicht mit der hautpflegenden Zusammensetzung behandelt wurde. Dieser Vorteil kann beispielsweise den Transfer der hautpflegenden Verbindung vom Textil auf die Haut, einen geringeren Wassertransfer von der Haut auf das Textil oder eine geringere Reibung auf der Hautoberfläche durch das Textil umfassen. Geeignete hautpflegende Verbindungen umfassen beispielsweise Wachse, Pflanzenextrakte, höhere Fettsäuren, höhere Fettalkohole, Ester, Kohlenwasserstoffe, Lipide, Vitamine, Sonnenschutzmittel oder Phospholipide.

Die Waschmittelzusammensetzung und die weitere Zusammensetzung können in einer Ausführungsform zumindest teilweise überlappend auf dem textilen Substrat angeordnet sein. In einer alternativen Ausführungsform sind die Waschmittelzusammensetzung und die weitere Zusammensetzung nebeneinander auf dem textilen Substrat angeordnet.

In einer bevorzugten Ausführungsform weist die Waschmittelzusammensetzung eine Farbe auf, die von der Farbe der weiteren Zusammensetzung verschieden ist.

In verschiedenen Ausführungsformen muss nicht die gesamte Oberfläche des textilen Substrats mit der verfestigten Waschmittelzusammensetzung und der gegebenenfalls vorhandenen weiteren Zusammensetzung bedeckt sein, solange die Waschmittelzusammensetzung in mindestens einem Teil der Substratoberfläche, einer Zone, auf dem Substrat vorliegt.

Die Waschmittelzusammensetzung und die gegebenenfalls vorliegenden weiteren Zusammensetzungen können auf eine oder beide Seiten des textilen Substrats aufgebracht werden. Vorzugsweise werden die Waschmittelzusammensetzung und die gegebenenfalls vorliegenden weiteren Zusammensetzungen auf einer Seite des textilen Substrats aufgebracht. Dies hat insbesondere den Vorteil, dass der Verbraucher beim Dosieren des Waschartikels nicht in direkten Kontakt mit den Zusammensetzungen kommen muss, sondern den Waschartikel auf der Zusammensetzungs-freien Oberfläche problemlos anfassen kann.

Es ist möglich, die Waschmittelzusammensetzung als flüssige Lösung, Aufschlämmung oder als Paste auf das textile Substrat aufzubringen und anschließend einen Trocknungsschritt durchzuführen und dabei das Aufschlämmungs- beziehungsweise Lösungsmittel, beispielsweise Wasser, so weit zu entfernen, dass sich die Waschmittelzusammensetzung auf dem textilen Substrat durch Trocknen verfestigt. Bevorzugt ist jedoch, die Waschmittelzusammensetzung als erwärmte Schmelze auf das textile Substrat aufzubringen und diese anschließend durch Abkühlen zu verfestigen. Dabei muss nicht die gesamte Waschmittelzusammensetzung durch Schmelzen verflüssigt vorliegen, es ist im Gegenteil möglich, dass die Schmelze teilchenförmige Feststoffe, wie beispielsweise anorganische Builder enthält.

### Beispiel 1

Es wurden Stammlösungen eines Polyorganosiloxans (DOW Corning AC-8066) in Wasser hergestellt (5, 10 und 20 Gew.-%). Diese wurden jeweils auf drei Bechergläser verteilt und es wurden Gewebeproben (Viskose/Lyocell (70/30) bzw. Viskose/Polymilchsäure (PLA) (70/30), jeweils 5 cm x 5 cm) für 20 Minuten in die Lösungen eingetaucht. Insgesamt wurden für jede Stammlösung sechs Gewebeproben verwendet (Dreifachbestimmung). Die Gewebeproben wurden über Nacht auf einem Filterbogen getrocknet.

Die Schmutzlösung (FI-Schmutz SBL-2004) für die nachfolgenden Waschversuche wurde nach folgendem Verfahren hergestellt: 0,85 g FI-Schmutz wurden in 6.000 g Wasser eingebracht und ca. 1,5 Stunden bei erhöhter Temperatur (Rührplatte: 68°C) gerührt.

Auf Mehrfachrührplatten wurden Bechergläser bereitgestellt und anschließend mit je 200 mL der Schmutzlösung befüllt. Die ausgerüsteten Gewebeproben sowie Blindproben ohne Ausrüstung wurden anschließend jeweils einzeln in ein Becherglas gegeben. Danach wurde die Rührfunktion eingeschaltet und für 1 Stunde bei Raumtemperatur konstant gerührt. Nach dem Herausnehmen der Proben wurde diese über Nacht zum Trocknen aufgehangen.

Die Beurteilung erfolgte visuell von 3 Personen, indem die ausgerüstete Gewebeprobe mit der dazugehörigen Blindprobe verglichen wurde.

**Tabelle 1: Schmutzfangversuche mit Tüchern, welche mit 5, 10 und 20%igen Lösung behandelt wurden und unbehandelten Tüchern als Vergleich. Je höher der Wert, desto höher der Vergrauungsgrad der Stoffprobe → Vergrauungsinhibierende Wirkung auf die Wäsche. 0 = unbehandelte Stoffprobe vor dem Waschversuch**

| | 5%ige Lösung | Vergleich | 10%ige Lösung | Vergleich | 20%ige Lösung | Vergleich |
|---|---|---|---|---|---|---|
| Viskose/PLA | 2 | 1,6 | 1,6 | 1,4 | 2,3 | 1,6 |
| Viskose/Lyocell | 1,6 | 1 | 1,7 | 1,4 | | |

## Patentansprüche

1. Beschichteter textiler Waschartikel, umfassend ein wasserunlösliches textiles Substrat, wobei das textile Substrat mit mindestens einem modifizierten oder unmodifizierten Polyorganosiloxan und/oder mindestens einem amorphen Siliziumdioxid beaufschlagt ist.

2. Beschichteter textiler Waschartikel gemäß Anspruch 1, **dadurch gekennzeichnet, dass** das
(1) mindestens eine modifizierte oder unmodifizierte Polyorganosiloxan ausgewählt ist aus der Gruppe von Polyalkylsiloxanen, Fluorsiliconen, Ether modifizierten Siliconen und Polyether modifizierten Siliconen, und/oder
(2) mindestens eine modifizierte oder unmodifizierte Polyorganosiloxan ein Molekulargewicht von 150 bis 50.000 g/mol oder 1.000 bis 35.000 g/mol oder 2.500 bis 25.000 g/mol aufweist, und/oder
(3) mindestens eine modifizierte oder unmodifizierte Polyorganosiloxan in 0,001 bis 50 Gew.-% oder 1,0 bis 10,0 Gew.-%, bezogen auf das Gesamtgewicht des beschichteten textilen Waschartikels, enthalten ist.

3. Beschichteter textiler Waschartikel gemäß einem der vorstehenden Ansprüchen, **dadurch gekennzeichnet, dass** der Waschartikel zudem eine Waschmittelzusammensetzung, die verfestigt auf dem textilen Substrat vorliegt, umfasst.

4. Beschichteter textiler Waschartikel gemäß einem der vorstehenden Ansprüchen, **dadurch gekennzeichnet, dass** das textile Substrat Fasern aufweist, die mit einer Wirkstoff-haltigen, wasserlöslichen Beschichtung versehen sind.

5. Beschichteter textiler Waschartikel gemäß dem vorstehenden Anspruch, **dadurch gekennzeichnet, dass** die wasserlösliche Beschichtung aus einem schichtbildenden Material gebildet ist, welches ausgewählt ist aus der Gruppe umfassend partikuläre Sole und wasserlösliche Polymere.

6. Beschichteter textiler Waschartikel gemäß einem der vorstehenden Ansprüchen, **dadurch gekennzeichnet, dass** das textile Substrat mit Mikrokapseln, die mindestens einen Wirkstoff enthalten, beaufschlagt ist.

7. Beschichteter textiler Waschartikel gemäß Anspruch 6, **dadurch gekennzeichnet, dass** die Mikrokapseln mittels eines auf dem textilen Substrat applizierten Klebstoffs an dem textilen Substrat befestigt sind.

8. Beschichteter textiler Waschartikel gemäß einem der vorstehenden Ansprüchen, **dadurch gekennzeichnet, dass** das textile Substrat
(1) ein Gewebe oder ein Vlies ist, und/oder
(2) aus Cellulose, Polyester, Polyethylen, Polypropylen, Polyethylenterephthalat, Polybutylenterephthalat, Rayon, Polyacryl, Polymilchsäure, Lyocell, Viskose oder Nylon oder Mischungen davon besteht.

9. Beschichteter textiler Waschartikel gemäß einem der vorstehenden Ansprüchen, **dadurch gekennzeichnet, dass** auf dem Substrat wenigstens eine Waschmittelzusammensetzung und eine weitere Zusammensetzung verfestigt vorliegt.

10. Beschichteter textiler Waschartikel gemäß Anspruch 9, **dadurch gekennzeichnet, dass** die wenigstens eine weitere Zusammensetzung ausgewählt ist aus der Gruppe der Textil- und/oder Haut-pflegenden Zusammensetzungen.

11. Beschichteter textiler Waschartikel gemäß einem der Ansprüche 9 und 10, **dadurch gekennzeichnet, dass** die Waschmittelzusammensetzung und die weitere Zusammensetzung nebeneinander auf dem textilen Substrat angeordnet sind.

12. Beschichteter textiler Waschartikel gemäß einem der Ansprüche 9 und 10, **dadurch gekennzeichnet, dass** die Waschmittelzusammensetzung und die weitere Zusammensetzung zumindest teilweise überlappend auf dem textilen Substrat angeordnet sind.

13. Verwendung eines beschichteten textilen Waschartikels gemäß einem der Ansprüche 1 bis 12 zum Waschen von Wäsche und/oder zur Unterbindung von Vergrauung von Wäsche.

14. Verfahren zum maschinellen oder manuellen Waschen von Wäsche unter Einsatz eines textilen Waschartikels gemäß einem der Ansprüche 1 bis 12.

15. Verfahren zur Herstellung eines beschichteten textilen Waschartikels nach einem der Ansprüche 1 bis 12, umfassend die Schritte:
(i) Bereitstellen eines wasserunlöslichen textilen Substrats,
(ii) Beaufschlagen des textilen Substrats mit mindestens einem modifizierten oder unmodifizierten Polyorganosiloxan und/oder mit mindestens einem amorphen Siliziumdioxid und gegebenenfalls einer bei Erwärmung über 30°C fließfähigen Waschmittelzusammensetzung und gegebenenfalls einer weiteren Zusammensetzung auf mindestens einer Zone des textilen Substrats und
(iii) Abkühlen und Erhärten der Komponenten auf dem textilen Substrat.
